# EUROPEAN PATENT APPLICATION

(11) **EP 4 375 644 A1**
(43) Date of publication of application: **29.05.2024**
(21) Application number: 22208827.0
(22) Date of filing: 22.11.2022
(51) Int. Cl.: G01N 21/64, G01J 3/44, C12Q 1/6869

(54) **FINGERPRINT ANALYSIS OF SINGLE MOLECULE EVENTS**

(71) Applicant: Gnothis Holding AG, 6330 Cham (CH)
(72) Inventor: EDMAN, Lars, 6330 Cham (CH); LUNDBERG, Oscar, 6330 Cham (CH); REDIN, David, 6330 Cham (CH)
(74) Representative: Weickmann & Weickmann PartmbB

(57) **Abstract**

The present disclosure relates to the analysis of molecular events as observed by irradiating a sample with a primary electromagnetic radiation, and detecting secondary electromagnetic radiation emitted by the sample induced by said primary electromagnetic radiation, wherein the sample is irradiated by a primary electromagnetic radiation and wherein at least one characteristic of said primary electromagnetic radiation is altered during the time period of the event.

## Description

The present disclosure relates to the analysis of molecular events as observed by irradiating a sample with a primary electromagnetic radiation, and detecting secondary electromagnetic radiation emitted by the sample induced by said primary electromagnetic radiation, wherein the sample is irradiated by a primary electromagnetic radiation and wherein at least one characteristic of said primary electromagnetic radiation is altered during the time period of the event.

### Background

Sequencing of the human genome or the genome of other organisms and the determination and comparison of individual sequence variants requires the provision of sequencing methods which firstly are fast and secondly can be employed routinely and cost-effectively.

The high demand for cost-efficient sequencing has driven the development of high-throughput sequencing technologies that parallelize the sequencing process producing a plurality of sequences concurrently. Examples of these sequencing technologies are massively parallel signature sequencing (Lynx Therapeutics), polony sequencing (Life Technologies), 454 pyrosequencing (Roche Diagnostics), illumina sequencing (Solexa Inc.), sequencing by ligation (Life Technologies), ion torrent semiconductor sequencing (Life Technologies) or DNA nanoball sequencing (Complete Genomics). These technologies allow rapid analysis of a consensus sequence in a nucleic acid population. Mutations existing in minority sequences in the nucleic acid population to be analysed, e.g., in a minority of cellular genomes, however, will not be detected since they are obscured by the majority of other sequences present in the population.

In order to address this issue, single molecule sequencing processes in several different formats have been developed. Several of these processes are within the field of fluorescence spectroscopy (FCS) and involve detection and analysis of single molecules by fluorescence. Typically, nucleic acid-polymerizing enzymes and/or nucleic acid-degrading enzymes and fluorescence-labelled nucleic acids and/or nucleotide building blocks are used for individually determining the sequence of a single nucleic acid molecule on the basis of a time-dependent change in fluorescence when nucleotide building blocks are incorporated into or cleaved off from a nucleic acid molecule. Single molecule sequencing processes and devices adapted for performing such processes are e.g., described in co-owned applications WO 2002/097406, WO 2003/052137, WO 2006/013110, WO 2013/131888, WO 2015/104245, WO 2017/001407, and WO 2018/104301.

In known processes for analyzing single molecule events, a sample comprising components of the single molecule event is irradiated with electromagnetic radiation that is constant over time. The detected fluorescence, however, will change in time as the single molecule event proceeds. In the example of a single DNA or RNA polymerase incorporating fluorescently labelled nucleotides, the detected signal will change in real time as labelled nucleotides are incorporated. If each of the four bases A, T, C, and G of a DNA molecule, or A, U, C, and G of an RNA molecule are attached with moieties that have unique fluorescence properties for each base/nucleotide, it is in principle possible to detect which base that is incorporated by detecting its unique fluorescence signature (c.f. US 7,056,661 B2 the content of which is herein incorporated by reference).

In reality, the detected emission wavelength distributions of fluorescently labelled nucleotides will have certain overlaps, which makes it impossible, even theoretically, to provide a 100% accurate determination of all incorporated nucleotides.

It was one of several objects of the present disclosure to provide improved means of distinguishing between those overlapping fluorescence emission spectra of labelled components taking part in a single molecule event.

### Summary of the Invention

In a first aspect, the present disclosure relates to a method for analyzing a single molecule event comprising:
- emitting a primary electromagnetic radiation from a radiation source to a sample where a single molecule event takes place, wherein said primary radiation causes emission of a secondary electromagnetic radiation from a component of said single molecule event, and wherein said secondary electromagnetic radiation is distinguishable from said primary electromagnetic radiation, and
- detecting said secondary electromagnetic radiation,
wherein the method further comprises altering at least one characteristic of said primary electromagnetic radiation during the time period of the single molecule event, whereby primary electromagnetic radiation having a first characteristic and primary electromagnetic radiation having at least one further characteristic is emitted to said sample during said time period wherein said first characteristic is different from said at least one further characteristic.

In a particular embodiment, the single molecule event comprises a sequence analysis of a single nucleic acid molecule.

A further aspect relates to a device for analyzing a single molecule event comprising:
- means for providing at least one sample adapted for a single molecule event,
- a radiation source adapted for emitting a primary electromagnetic radiation to said sample, wherein said primary radiation causes emission of a secondary electromagnetic radiation from a component of said single molecule event, and wherein said secondary electromagnetic radiation is distinguishable from said primary electromagnetic radiation, and
- means for detecting said secondary electromagnetic radiation,
wherein the radiation source is adapted for altering at least one characteristic of said primary electromagnetic radiation at least once during the time period of the single molecule event, whereby primary electromagnetic radiation having a first characteristic and primary electromagnetic radiation having at least one further characteristic is emitted to said sample during said time period wherein said first characteristic is different from said at least one further characteristic.

In a particular embodiment, the device is adapted for the sequence analysis of a single nucleic acid molecule.

A further aspect relates to the use of the above method or the above device for providing an increased accuracy in the analysis of a single molecule event.

In a particular embodiment, the use is for providing an improved distinction between different incorporated nucleobases during a polymerase reaction.

### Items of the Specification

1. A method for analyzing a single molecule event comprising:
   - emitting a primary electromagnetic radiation from a radiation source to a sample where a single molecule event takes place, wherein said primary radiation causes emission of a secondary electromagnetic radiation from a component of said single molecule event, and wherein said secondary electromagnetic radiation is distinguishable from said primary electromagnetic radiation, and
   - detecting said secondary electromagnetic radiation,
   wherein the method further comprises altering at least one characteristic of said primary electromagnetic radiation during the time period of the single molecule event, whereby primary electromagnetic radiation having a first characteristic and primary electromagnetic radiation having at least one further characteristic is emitted to said sample during said time period wherein said first characteristic is different from said at least one further characteristic
2. The method of item 1, wherein said secondary electromagnetic radiation is separately detected for said first characteristic and for said at least one further characteristic of said primary electromagnetic radiation.
3. The method of item 2, wherein a combined signal of said secondary electromagnetic radiation for said first characteristic and for said at least one further characteristic of said primary electromagnetic radiation is provided and analyzed.
4. The method of item 3, wherein said combined signal is a fingerprint from n different characteristics of said primary electromagnetic radiation, wherein n is particularly 2, 3, or 4.
5. The method of any one of the preceding items, wherein at least one characteristic of the primary electromagnetic radiation is altered between at least 2 different states during the time period of the single molecule event.
6. The method of item 5, wherein at least one characteristic of the primary electromagnetic radiation is altered between about 2-10 different states during the time period of the single molecule event.
7. The method of any one of items 5-6, the intervals of one or more individual states of the primary electromagnetic radiation are selectively varied, e.g., increased compared to other individual states.
8. The method of any one of the preceding items, wherein said radiation source comprises at least one laser, particularly a plurality of lasers having different emission wavelengths.
9. The method of any one of the preceding items, wherein a plurality of individual samples is provided, e.g., at least 10, at least 100, at least 1,000 or at least 10,000 individual samples.
10. The method of item 9, wherein said primary electromagnetic radiation from the radiation source is split into a plurality of separate radiation beams, e.g., by a diffractive optical element.
11. The method of item 10, wherein separate radiation beams are emitted into individual samples or groups of individual samples.
12. The method of any one of the preceding items, wherein said at least one sample comprises at least one sample spot on a support.
13. The method of item 12, wherein said support comprises a substrate and a plurality of sample spots on the surface of the support, wherein the sample spots are spatially separated from another.
14. The method of item 12 or 13, wherein a component of said single molecule event is immobilized to said at least one sample spot.
15. The method of item 14, wherein said immobilized component comprises a biological moiety, e.g., a biomolecule.
16. The method of item 15, wherein said immobilized reaction component comprises a nucleic acid-polymerizing enzyme, particularly a DNA or RNA polymerase.
17. The method of any one of the preceding items, wherein said at least one sample comprises at least one luminescent component, particularly at least one fluorescent component, of the single molecule event, e.g., a reactant, reaction intermediate and/or reaction product, wherein said at least one luminescent component emits said secondary electromagnetic radiation.
18. The method of item 17, wherein the at least one luminescent component is compound comprising a luminescent group, e.g., a luminescent label group, particularly a compound comprising a fluorescent group, e.g., a fluorescent label group.
19. The method of item 17 or 18, wherein said sample comprises a plurality of different luminescent components, wherein at least some of said luminescent components have distinguishable and partially overlapping emission spectra of secondary electromagnetic radiation.
20. The method of any one of items 17-19, wherein said sample comprises a plurality of different luminescent components, particularly fluorescent components wherein at least some of said luminescent components, particularly fluorescent components have distinguishable and partially overlapping luminescence emission spectra, particularly distinguishable and partially overlapping fluorescence emission spectra.
21. The method of any of items 17-20, wherein said at least one luminescent component is present in said sample in free form.
22. The method of any one of the preceding items comprising separately detecting a plurality of single molecule events in different samples, particularly comprising separately detecting a plurality of single molecule events in different samples in parallel.
23. The method of any one of the preceding items comprising detecting multiple subsequent single molecule events in one sample, particularly comprising separately detecting multiple subsequent single molecule events in different samples in parallel.
24. The method of item 23, wherein said multiple single molecule events comprise subsequent nucleic acid elongation and/or degradation steps of a single molecule nucleic acid sequence analysis.
25. The method of item 24, wherein said single molecule nucleic acid sequence analysis comprises multiple steps of nucleic acid elongation, wherein a luminescent nucleotide building block, e.g., a luminescent nucleoside polyphosphate, e.g., comprising 3 to 15 phosphate groups is incorporated into a nucleic acid molecule in the presence of a nucleic acid-polymerizing enzyme, e.g., a DNA or RNA polymerase.
26. The method of item 25 comprising detecting secondary electromagnetic radiation from the incorporation of said luminescent nucleotide building block, e.g., said luminescent nucleoside polyphosphate into said nucleic acid molecule.
27. The method of any one of the preceding items comprising altering a characteristic of said primary electromagnetic radiation several times during the time period of the single molecule event.
28. The method of any one of the preceding items comprising altering a characteristic of said primary electromagnetic radiation during each of the time periods of several subsequent single molecule events.
29. The method of any one of the preceding items comprising wherein said altering comprises a temporary shutdown of said primary electromagnetic radiation during the time period of the single molecule event.
30. The method of any one of the preceding items comprising altering a characteristic of said primary electromagnetic radiation selected from the group:
   - wavelength,
   - amplitude,
   - pulsed operation,
   - polarization, and
   - a combination of two or more of said characteristics.
31. The method of any one of the preceding items comprising altering the wavelength of said primary electromagnetic radiation during said single molecule reaction.
32. The method of any one of the preceding items comprising altering the characteristic of said primary electromagnetic radiation in time intervals that are in the range of about 50 ns to about 10 s, about 1 µs to about 500 ms, or about 10 µs to about 100 ms.
33. The method of any one of the preceding items wherein the at least one characteristic of the primary electromagnetic radiation is controlled and optionally adjusted in accordance with the detected secondary electromagnetic radiation emitted from the sample of the single molecule event.
34. The method of item 33, wherein the control and optional adjustment is performed by a detector adapted for detecting the secondary electromagnetic radiation emitted from the sample of the single molecule event.
35. The method of item 33 or 34, wherein the control and optional adjustment is a dynamic process based on previous measurements of secondary radiation.
36. A device for analyzing a single molecule event comprising:
   - means for providing at least one sample adapted for a single molecule event,
   - a radiation source adapted for emitting a primary electromagnetic radiation to said sample, wherein said primary radiation causes emission of a secondary electromagnetic radiation from a component of said single molecule event, and wherein said secondary electromagnetic radiation is distinguishable from said primary electromagnetic radiation, and
   - means for detecting said secondary electromagnetic radiation,
   wherein the radiation source is adapted for altering at least one characteristic of said primary electromagnetic radiation at least once during the time period of the single molecule event, whereby primary electromagnetic radiation having a first characteristic and primary electromagnetic radiation having at least one further characteristic is emitted to said sample during said time period wherein said first characteristic is different from said at least one further characteristic.
37. The device of item 36 adapted for performing the method of any one of items 1-35.
38. The device of item 36 or 37 adapted for performing a single molecule nucleic acid sequence analysis.
39. Use of the method of any one of items 1-35 or the device of any one of items 36-38 for providing an increased accuracy in the analysis of a single molecule event.
40. The use of item 39 for providing an improved distinction between different incorporated nucleobases during a polymerase reaction.

### Detailed Description

The present disclosure provides methods and devices for analyzing a single molecule event, wherein the single molecule event is associated with the emission of an electromagnetic radiation from a sample, e.g., a sample spot on a support comprising the components of the single molecule event. In particular embodiments, the single molecule event encompasses a reaction of a biological moiety which is associated with emission of a characteristic electromagnetic radiation.

The present disclosure addresses the need of improved accuracy in the analysis of a single molecule event as observed by irradiating a sample with a primary electromagnetic radiation, and simultaneously detecting the resulting secondary electromagnetic radiation emitted by the sample as induced by said irradiation.

In particular, the present disclosure provides a device and process to improve accuracy in the determination of each kinetic step of a single molecule event. This is achieved by allowing the source that irradiates a sample with electromagnetic radiation to change in time during the observation of any single kinetic step of a single-molecule reaction.

For this purpose, at least one characteristic of the primary radiation is altered during the time period of the single molecule event to be analyzed. Accordingly, primary electromagnetic radiation having a first characteristic and primary electromagnetic radiation having at least one further characteristic is emitted to the sample of the single molecule event during the time period when the single molecule event takes place. The first characteristic of the primary electromagnetic radiation is different from the at least one further characteristic. The characteristic may be selected, e.g., from wavelength, amplitude, pulsed operation, polarization, and a combination of two or more of said characteristics. Thus, the first wavelength (or any other characteristic or combination of characteristics) of the primary electromagnetic radiation is different from the at least one further wavelength (or any other characteristic or combination of characteristics).

According to certain embodiments, the secondary electromagnetic radiation emitted by a component associated with the single molecule event is separately detected for the first characteristic and for the at least one further characteristic of the primary electromagnetic radiation. In particular embodiments, a combined signal of the secondary electromagnetic radiation is provided. This combined signal is based on the secondary electromagnetic radiation signal induced by the first characteristic of the primary electromagnetic radiation and on the secondary electromagnetic radiation signal induced by the at least one further characteristic of the primary electromagnetic radiation. By analyzing the combined signal, the accuracy of detection is substantially improved.

In particular embodiments, the combined signal is a fingerprint from n different characteristics of said primary electromagnetic radiation, wherein n is particularly 2, 3, or 4. The combined signal may be depicted a n-dimensional diagram or plot, wherein n is an integer of at least 2.

In an exemplary embodiment, a single molecule DNA sequencing procedure is performed. In particular, a polymerization reaction catalyzed by a DNA or RNA polymerase takes place, wherein a DNA or RNA molecule binds to a single polymerase molecule, and nucleotide building blocks are incorporated one-by-one thus elongating the DNA or RNA molecule. The irradiation source that irradiates a sample with electromagnetic radiation is altered in at least one characteristic once or several times during the time period when each nucleotide is incorporated into the DNA or RNA strand.

In certain embodiments, a characteristic of the primary electromagnetic radiation is altered several times, e.g., 2, 3, 4, 5, or more times, e.g., up to 100 times during the time period of the single molecule event.

When analyzing multiple single molecule events, a characteristic of the primary electromagnetic radiation may be altered during several consecutive single molecule events. In those embodiments, the alteration scheme may be the same for each of the multiple consecutive single molecule events or it may differ between the multiple consecutive single molecule events. In certain embodiments, the alteration may a temporary shutdown of the primary electromagnetic radiation during the time period of the single molecule event, i.e., the sample is irradiated with primary electromagnetic radiation only in certain intervals during the time period of the single molecule event.

In certain embodiments, the characteristic of the primary electromagnetic radiation to be altered is selected from the group:
- wavelength,
- amplitude,
- pulsed operation,
- polarization, and
- a combination of two or more of said characteristics.

In certain embodiments, the wavelength of the primary electromagnetic radiation is altered at least once during the time period of the single molecule event.

According to the present disclosure, at least one characteristic of the primary electromagnetic radiation is altered between at least 2 different states during the time period of the single molecule event, e.g., between at least 2 different wavelengths or wavelength combinations. In certain embodiments, a characteristic of the primary electromagnetic radiation is altered between about 2-10 , e.g., 2, 3, 4, 5, 6, 7, 8, 9, or 10 different states during the time period of the single molecule event.

According to the present disclosure, at least one characteristic of the primary electromagnetic radiation is altered at least once during the time period of a single molecule event. In certain embodiments, the at least one characteristic of the primary electromagnetic radiation is altered in time intervals that are in the range of about 50 ns to about 10 s, about 1 µs to about 500 ms, or about 10 µs to about 100 ms.

In certain embodiments, at least one characteristic of the primary electromagnetic radiation is controlled and optionally adjusted in accordance with the detected secondary electromagnetic radiation emitted from the sample of the single molecule event. In certain embodiments, the control and optional adjustment is performed by a detector adapted for detecting the secondary electromagnetic radiation emitted from the sample of the single molecule event. In particular embodiments, the control and optional adjustment is a dynamic process based on previous measurements of secondary radiation. In particular embodiments, the control and optional adjustment is based on a real-time analysis of the detected secondary electromagnetic radiation. This control and optional adjustment may provide a feedback mechanism for optimizing the scheme of altering the at least one characteristic of the primary electromagnetic radiation.

In certain embodiments, the detector (optionally in combination with suitable hardware and/or software) provides instructions for the alteration of the primary radiation source in accordance with a scheme depending on the type of molecular event that is currently analyzed, e.g., as evidenced by a previously measured secondary radiation in the analysis procedure of the event.

In certain embodiments, the detector (optionally in combination with suitable hardware and/or software) provides instructions for the alteration of the primary radiation source in accordance with a scheme depending on general conditions, e.g., temperature, type and concentration of reagents etc., e.g., as evidenced by the measured secondary radiation in the analysis procedure of one or several previous events.

In a specific embodiment, the detector (optionally in combination with suitable hardware and/or software) may instruct the primary radiation source to become completely inactive for a predetermined interval during the time period of the single molecule event. This instruction may be provided based on analysis of initially measured secondary radiation.

In further embodiments, the changes in the primary electromagnetic radiation are deterministic without the detector analyzing the secondary electromagnetic radiation in real time.

In particular embodiments, the wavelength of the primary electromagnetic radiation is altered several times during each said incorporation event in a single molecule nucleic acid synthesis procedure. Each A, C, T/U, and G nucleotide building block is differently labeled, e.g., is chemically attached to a different fluorescent moiety each having an emission spectrum that has a unique maximum, but wherein each emission spectrum has a distribution around its maximum that makes the distributions overlap. In such a case, a radiation source capable of emitting a plurality of different wavelengths, e.g., a set of lasers with a specified unique output wavelength may be provided. Thereby, the wavelength of the primary electromagnetic radiation emitted by the radiation source may be altered between several different states during the time period of the single molecule event, e.g., the incorporation of a nucleotide building block into the elongated nucleic acid molecule. For example, in a first state, a first laser is turned on whereas the rest of the lasers are turned off, in a second state, a second laser is turned whereas the rest of the lasers are turned off and so on. The alteration between different states is a sequential process that is rapid enough so that several rounds of excitation of each laser can be carried out during each single event, e.g., several times during each incorporation event. Thereby it is possible to record a signal generated by the secondary radiation when each laser is turned on several times during an incorporation event by a detector. The detector is synchronized with the lasers and is consequently aware of when which laser is activated. This, in turn, allows to provide a specific response curve for each laser wavelength.

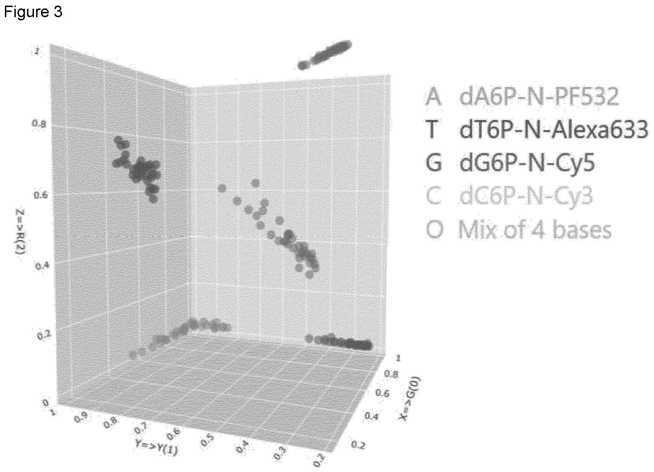

These specific response curves for each event may be combined to obtain a multidimensional response plot or diagram for each event. Accordingly, a lack of accuracy associated with overlapping emission distributions of individual labeled components can be overcome by providing a combined signal of the collected emissions recorded for each laser during one incorporation event. This combined signal may be presented, in the general case as an n-dimensional plot or diagram, or "fingerprint. In an exemplary, but not limiting embodiment, wherein three different lasers are used (n=3), a three-dimensional fingerprint may be provides as shown in Fig. 3 infra. According to this fingerprint, there is no longer any overlap between the emission spectra of the labelled nucleotide building blocks and each labelled nucleotide is clearly detected within a certain space of the three-dimensional plot.

In further exemplary embodiments, the number of lasers may be increased from n=3 to a larger number, e.g., 4, 5 or even more, causing a further separation of the different signals within the n-dimensional fingerprint.

Corresponding fingerprints may also be provided by altering other characteristics or combinations of several characteristics of the primary electromagnetic radiation.

Moreover, the intervals of one or more individual states of the primary electromagnetic radiation may be selectively varied, e.g., increased, compared to other individual states. For example, depending on the emission characteristics of a luminescent component, the length of one or several active periods for a selected wavelength used for the primary electromagnetic radiation may be varied, e.g., increased compared to active periods of other wavelengths.

In certain embodiments, introduction of periods of zero primary electromagnetic radiation may be used between active periods, e.g., different active periods.

In certain embodiments, the individual states of the primary electromagnetic radiation may comprise combinations of different wavelengths as well as their relative amplitudes. In certain embodiments, the individual states of the primary electromagnetic radiation may comprise combinations of different wavelengths and polarizations.

The sample of the single molecule event typically comprises a sample spot on a support. The support may comprise a substrate and a plurality of sample spots on the support surface that are spatially separated from another. In certain embodiments, the support surface is formed by the substrate and the sample spots. In certain embodiments, the substrate forms a contiguous area in which the sample spots are distributed. An individual sample spot on the support surface is surrounded by the substrate, which differs from the sample spot, e.g., in respect of the material and/or the surface. Typically, the substrate is adapted for inhibiting and/or blocking adhesion of biomolecules such as polypeptides whereas the sample spots are adapted for allowing adhesion of desired biomolecules.

On a sample spot, a component taking part in the single molecule event, e.g., a biological moiety is immobilized. In certain embodiments, the component comprises a biomolecule, particularly a single biomolecule. The term "single biomolecule" encompasses a single molecular entity such as a polypeptide or a complex consisting of a plurality of individual units, e.g., individual molecular entities wherein the individual units form together a functional biological moiety.

In certain embodiments, the support is a substantially planar support, i.e., it does not comprise elevations or recesses of about 1000 nm or more or of about 100 nm or more. In further embodiments, the support is a structured support, e.g., a support comprising recesses such as wells that may have a volume of about 5 × 10⁻²⁴ liters to about 1 × 10⁻¹⁵ liter, or pillars of height of about 1 nm to 500 nm. In principle, the support may have any design, as long as a reaction space can be formed which enables the occurrence of a single molecule event on said at least two sample spots where the single biomolecules is immobilized.

In certain embodiments, the substrate is an optically transparent material, i.e., a material that is substantially transparent for electromagnetic radiation, e.g., radiation in the visible range and/or radiation in the near-infrared range. In certain embodiments, the substrate comprises a material having an absolute refractive index of at least 1.01, e.g., about 1.5 to about 3 in the visible range or about 1.5 to about 4 in the near-infrared range. In further embodiments, the substrate is an optically opaque material, e.g., a metal or semi-metal such as silicon.

In particular embodiments, the substrate comprises a non-electrically conductive material. Specific examples are glass, quartz, plastic, metal oxide-based materials, e.g., silicon dioxide-based materials such as glass, silica, or quartz, or composites comprising said materials. In further embodiments, the substrate comprises an electrically conductive material, e.g., an optically transparent material such as indium tin oxide.

Typically, the substrate has a thickness of about 10 µm to about 5 mm, particularly about 20 µm to about 2 mm.

The surface of the support comprises a plurality of sample spots that are spatially separated from another by the substrate surface. The sample spots are adapted for attachment of biomolecules. In certain embodiments, the support comprises a plurality of sample spots, e.g., at least 10, at least 100, at least 1,000, at least 10,000 sample spots, or at least 100,000 spots. In certain embodiments, the support may comprise up 10⁶ or 10⁹ or even more sample spots.

In certain embodiments, a sample spot comprises or consists of at least one electrically conductive material, e.g., a metal including a single metal or a combination of metals, e.g., an alloy or mixture of a plurality of different metals. For example, a metal that is capable of attachment to a sulfur containing moiety, e.g., in the form of a thiol or a disulfide, or a metal that is capable of attachment to a chelating moiety, e.g., a poly-histidine tag, is suitable. In certain embodiments, the metal has a positive electrochemical potential. Specific examples of suitable metals include but are not limited to Au, Cu, Ni, Pt, Pd, Rh, Ir, Os, Ru, and any combination thereof comprising at least two of said metals.

In certain embodiments, a sample spot comprises or consists of at least one at least one metal oxide including a single metal oxide or a combination of metal oxides. For example, a metal oxide that is capable of attachment to a phosphorus containing moiety, e.g., in the form of a phosphonic acid or phosphonic acid ester, or a metal oxide that is capable of attachment to a chelating moiety, e.g., a poly-histidine tag, is suitable. Specific examples of suitable metal oxides include TiO₂ and NiO.

In further embodiments, a sample spot comprises or consists of at least one non-electrically conductive material.

Sample spots may be prepared by vapour deposition of metals, which are vaporized on the support covered by a grid mask, which may be produced by electron beam lithography or equivalent technologies. The size of holes in the grid mask may correspond to the size of the spots on the support surface. Alternatively, the spots on the support may be prepared by site-specific deposition of nanoparticles, e.g., having a size of 2-10 nm, by precision pipetting of particles on the support, particularly on a support having a planar surface.

In particular embodiments, a sample spot has a size that is suitable for the attachment of a single biomolecule. In those embodiments, a sample spot has a diameter of about 1 nm to about 30 nm, particularly a diameter of about 2 nm to about 20 nm.

In certain embodiments, the sample spot is a separate object on the surface of the substrate. In certain embodiments, the sample spot has a lower face proximal to the substrate and an upper face distal to the substrate, wherein the distance between the lower and upper face defines the height of the sample spot. In particular embodiments, the height is about 50 pm to about 500 nm, particularly about 100 pm to about 20 nm, and more particularly about 500 pm to about 10 nm, e.g., about 2 nm.

The sample spots on the support are adapted for the attachment of a biomolecule, e.g., by a covalent or non-covalent attachment. The biomolecule may be selected from polypeptides, nucleic acids, carbohydrates, and any combination thereof, e.g., a glycosylated polypeptide, or a ribonucleoprotein. In certain embodiments, the biomolecule is a complex consisting of several individual units, e.g., several polypeptide units, or several polypeptide and nucleic acid units.

In specific embodiments, the biomolecule is a nucleic acid-polymerizing enzyme, particularly a DNA polymerase or an RNA polymerase, or a nucleic acid-polymerizing molecule complex, particularly a DNA- or RNA-polymerizing complex comprising a nucleic acid-polymerizing enzyme and a nucleic acid molecule. In particular embodiments, the biomolecule is a DNA polymerase having a DNA binding cleft, particularly a Family A DNA polymerase including, but not limited to a Klenow, Taq or T7 DNA polymerase or any genetically modified version thereof, or a Family B polymerase including, but not limited to a therminator, Phi29, RB-69 or T4 DNA polymerase or any genetically modified version thereof. Reference is made in this context to US 7,745,116 B2, the content of which is herein incorporated by reference.

In further embodiments, the biomolecule is a nucleic acid-degrading enzyme, particularly an exonuclease, or a nucleic acid-degrading molecule complex, particularly a DNA- or RNA-degrading molecule complex comprising a nucleic acid-degrading enzyme and a nucleic acid molecule.

In still further embodiments, the biomolecule is a gene editing enzyme, particularly a Cas nuclease such as a Cas3, Cas9, Cas10, or Cas12 nuclease or any genetically modified version thereof, e.g., a Cas nickase, or a gene editing complex comprising a gene editing enzyme and nucleic acid molecule, e.g., a guide RNA and/or a target nucleic acid.

The single molecule event to be detected takes place in a sample that comprises the required components of the event, for example, a biomolecule and optionally small molecules. At least one of the components is a luminescent component that comprises a luminescent group, i.e., a group that is capable of emitting a secondary electromagnetic luminescence radiation in response of being irradiated by the primary electromagnetic radiation. In certain embodiments, the luminescent component is a compound carrying a luminescent labeling group. In certain embodiments, the luminescent component is a compound that is capable of luminescence per se.

In certain embodiments, the luminescent component of the single molecule event may be a reactant, reaction intermediate and/or reaction product. In particular embodiments, the luminescent component is a fluorescent component, i.e., a component that is capable of emitting fluorescence radiation in response of being irradiated by the primary electromagnetic radiation. In certain embodiments, the fluorescent component is a compound carrying a fluorescent labeling group. In certain embodiments, the fluorescent component is a compound that is capable of fluorescence per se.

In particular embodiments, the sample of the single molecule event comprises a plurality of different luminescent components, wherein at least some of said luminescent components have partially overlapping emission spectra of secondary electromagnetic radiation. For example, the sample may comprise a plurality of different fluorescent components wherein at least some of said fluorescent components have distinguishable and partially overlapping fluorescence emission spectra.

The at least one luminescent component may be present in the sample in immobilized form and/or free form. In certain embodiments, the least one luminescent component is present in free form.

The method of the present disclosure may comprise separately analyzing a plurality of single molecule events in different samples, particularly in different sample spots on a support. In certain embodiments, the method comprises separately analyzing a plurality of single molecule events in different samples in parallel, particularly in different sample spots on a support.

In certain embodiments, the method of the present disclosure comprising analyzing multiple subsequent single molecule events in one sample, particularly comprising separately analyzing multiple subsequent single molecule events in different samples in parallel. The term "multiple single molecule events" encompasses a sequence of consecutive single molecule events that take place at the same sample, e.g., at the sample spot on a support. In certain embodiments, the sequence of consecutive single molecule events comprises up to 10, up to 100, up to 1,000, up to 10,000 or even more individual single molecule events, e.g., up to about 10,000,000 or 100,000,000 individual single molecule events. For example, multiple single molecule events may comprise subsequent nucleic acid elongation and/or degradation steps of a single molecule nucleic acid sequence analysis.

In particular embodiments, a single molecule nucleic acid sequence analysis comprises multiple steps of nucleic acid elongation, wherein a luminescent nucleotide building block, e.g., a luminescence-labeled nucleoside polyphosphate, e.g., comprising 3 to 15 phosphate groups is incorporated into a nucleic acid molecule in the presence of a nucleic acid-polymerizing enzyme, e.g., a DNA or RNA polymerase. Those embodiments may comprise detecting secondary electromagnetic radiation from the incorporation of a luminescent nucleotide building block, e.g., a luminescence-labeled nucleoside polyphosphate into said nucleic acid molecule.

According to the present disclosure, the sample at which the single molecule event takes place is irradiated with a primary electromagnetic radiation, wherein at least one characteristic of the primary electromagnetic radiation is altered during the time period of the single molecule event.

The present disclosure further provides a device for analyzing a single molecule event comprising:
- means for providing at least one sample adapted for a single molecule event,
- a radiation source adapted for emitting a primary electromagnetic radiation to said sample, wherein said primary radiation causes emission of a secondary electromagnetic radiation from a component of said single molecule event, and wherein said secondary electromagnetic radiation is distinguishable from said primary electromagnetic radiation, and
- means for detecting said secondary electromagnetic radiation,
wherein the radiation source is adapted for altering at least one characteristic of said primary electromagnetic radiation at least once during the time period of the single molecule event. Thereby primary electromagnetic radiation having a first characteristic and primary electromagnetic radiation having at least one further characteristic is emitted to the sample during the time period of the single molecule event wherein the first characteristic is different from the at least one further characteristic.

In certain embodiments, the device is adapted for performing the method as described above, e.g., for performing a single molecule nucleic acid sequence analysis.

The method and the device of the present disclosure are useful for providing an increased accuracy in the analysis of a single molecule event. In particular embodiments, they are useful for providing an improved distinction between different incorporated nucleobases during a polymerase reaction.

Methods and devices for analyzing single molecule events are e.g., disclosed in WO 2002/097406, WO 2003/052137, WO 2006/013110, WO 2013/131888, WO 2015/104245, WO 2017/001407, and WO 2018/104301, the contents of which are herein incorporated by reference.

For the analysis of a single molecule event, the biomolecules may be located at the sample spots on the support. There they are in contact with a sample liquid, which contains the free reaction partners. Thereby, one or more reaction spaces are defined. Particularly at least 100, at least 1000, or at least 10 000, and up to more than 10⁶ molecules may be analysed on a single support, e.g., a single planar support

A nucleic acid molecule whose sequence is to be determined may be selected, for example, from DNA molecules such as genomic DNA fragments, cDNA molecules, plasmids, etc., or else from RNA molecules such as mRNA molecules. The nucleic acid molecule may originate from genomic or expression libraries, generated from cells or organisms, e.g., eukaryotic or prokaryotic cells or organisms. This allows parallel sequencing of a plurality of different nucleic acid template molecules, e.g., at least 10, 100, 1.000 or 10.000 and up to 100.000, 10⁶ or 10⁷ or even more different nucleic acid molecules.

The nucleic acid molecules to be sequenced may be single-stranded nucleic acid molecules in a linear or circular form, e.g., in a covalently linked circular form. In order to obtain a circular nucleic acid template, a linear nucleic acid molecule may be subjected to a circularization procedure and optionally a strand-separation procedure during sample preparation. Circularization may be effected by ligation according to known protocols, e.g., using DNA or RNA ligases. In some embodiments, an adaptor and/or identifier molecule, i.e., a nucleic acid molecule of known sequence, may be coupled to the nucleic acid molecule.

The sequence determination may comprise nucleic acid elongation and/or nucleic acid degradation. The sequencing process includes one or more sequencing cycles.

The nucleic acid-synthesizing enzyme molecules are capable of elongating a primer annealed to a nucleic acid template molecule. Primer elongation may be carried out by progressively incorporating individual nucleotide building blocks at the 3'-terminus of a growing nucleic acid chain, wherein a nucleic acid molecule complementary to the sequence of the circular nucleic acid template is generated. The nucleic acid-synthesizing enzymes are selected from polymerases capable of a template specific nucleic acid polymerization, preferably from DNA polymerases and RNA polymerases, e.g., natural or modified polymerases, including thermostable DNA polymerases.

Specific examples of suitable DNA polymerases include Taq polymerases, exonuclease-deficient Taq polymerases, E.coli DNA polymerase I, Klenow fragment, reverse transcriptase, Φ29-related polymerases including wild-type Φ29 polymerase and derivatives of such polymerases, such as exonuclease-deficient forms, T7 DNA polymerase, T5 DNA polymerase, an RB69 polymerase and others.

Nucleic acid-degrading enzyme molecules are capable of progressively cleaving off individual nucleotide building blocks from a nucleic acid molecule. Preferably exonucleases, more preferably single-strand exonucleases which degrade in the 3'→5' direction or in the 5'→3' direction are used. Exonucleases which are particularly preferably used are 3'→5' exonucleases such as E.coli exonuclease I and E.coli exonuclease III, and 5'→3' exonucleases such as T7 exonuclease, E.coli exonuclease II and E.coli exonuclease VIII. Further, the exonuclease activities of various polymerases, e.g., the Klenow fragment, Taq polymerase or T4 polymerase may be used.

The nucleic acid-synthesizing enzyme molecules are contacted with a linear or circular nucleic acid template molecule, e.g., a single-stranded DNA or RNA molecule, and a primer molecule annealed to the nucleic acid template molecule or capable of annealing thereto. The primer molecule is preferably a single-stranded nucleic acid or nucleic acid analogue molecule having a free 3'-end which can be extended by an enzymatic reaction catalyzed by the immobilized nucleic acid-synthesizing enzyme molecules. The length of the primer molecule is selected to allow effective annealing to the template under reaction conditions. Usually, the length of the primer molecule is at least 8, at least 10, at least 12 or at least 15 nucleotides and e.g., up to 20, 25, 50 or 100 nucleotides, or even higher. In some embodiments, the primer is resistant against digestion by nucleic acid-degrading enzyme molecules, e.g., by incorporating nucleotide analogue building blocks and/or linkages between nucleotide building blocks, which are stable against degradation. In other embodiments, the primer is sensitive against digestion by nucleic acid-degrading enzyme molecules.

The sequence of the primer is selected in that it effectively anneals under reaction conditions to the template molecule. For instance, the primer may be a universal degenerated primer capable of statistically annealing to unknown nucleic acid sequences. In other embodiments, the primer may be capable of annealing to a known sequence portion of the nucleic acid template molecule. In this embodiment, a known adaptor and/or identifier sequence may be incorporated into the nucleic acid template molecule. The primer may be unlabelled or comprise fluorescent labelling groups.

Further, the presence of luminescent nucleotide building blocks, e.g., nucleotide building blocks carrying at least one fluorescent labelling group is required. Preferably, each different nucleotide building block (A, G, C, T/U) contains a different fluorescent labelling group.

The fluorescent labelling groups may be selected from known fluorescent labelling groups used for labelling biopolymers, particularly nucleic acids, such as, for example, fluorescein dyes, rhodamines, oxazines, for example Evoblue or Gnothis Blue, phycoerythrin, Cy3, Cy5, IR dyes or derivatives thereof, etc.

The nucleotide building blocks may carry (i) a fluorescence labelling group which remains with the building block when the building block is incorporated into a nucleic acid molecule during a primer elongation catalyzed by a nucleic acid-synthesizing enzyme molecule, and/or (ii) a fluorescence labelling group which is cleaved off from the building block when the building block is incorporated into a nucleic acid molecule during a primer elongation catalyzed by a nucleic acid-synthesizing enzyme molecule. Fluorescence labelling groups remaining with the building block are preferably attached to the α-phosphate group, to the sugar and/or to the nucleobase group.

In particular embodiments, fluorescence labelling groups remaining with the building block are attached to the nucleobase, e.g., via a linker which may have a chain-length of up to 15, preferably of 10-12 carbon atoms, optionally including heteroatoms, e.g., N, O or S atoms. Fluorescence labelling groups which are cleaved off when the building block is incorporated into a nucleic acid molecule may be attached to a terminal phosphate group, e.g., of a polyphosphate building block including, but not limited to a hexa-, penta-, tetra- or triphosphate building block such as the γ-phosphate group of a triphosphate building block. In certain embodiments, building blocks are selected which contain both (i) a fluorescence labelling group remaining after incorporation and (ii) a fluorescence labelling group cleaved off during incorporation. In this case, fluorescence groups capable of interacting with each other, e.g., by quenching and/or energy transfer, may be selected.

The nucleic acid molecules to be sequenced will contain fluorescent labelling groups in case the nucleic acid molecule is subjected to direct sequencing using a nucleic acid-degrading enzyme molecule. On the other hand, the nucleic acid molecule to be sequenced my not contain fluorescent labelling groups, if the nucleic acid molecule is used as a template in a primer elongation.

The sequencing procedure may involve a step of generating nucleic acid molecules having incorporated nucleotide building blocks in a primer elongation catalyzed by the nucleic acid-synthesizing enzyme molecules and/or a second step of cleaving off individual nucleotide building blocks from the generated nucleic acid molecules catalyzed by nucleic acid-degrading enzyme molecules. Dependent on the type of fluorescence labels, nucleic acid sequence determination may be carried out during primer elongation and/or during degradation.

Sequence determination during the primer elongation involves the use of nucleotide building blocks carrying a fluorescence-labelling group which is cleaved off from the building block when it is incorporated into a nucleic acid molecule. In this case, a time-dependent fluorescence change caused by cleaving off the fluorescence-labelling group from the nucleotide building block may be determined. Sequence determination during nucleic acid degradation involves the use of a nucleotide building block, which carries a fluorescence-labelling group which remains with the building block when it is incorporated into a nucleic acid molecule. Progressive cleavage of individual nucleotide building blocks from the nucleic acid molecules causes a time-dependent change of fluorescence when the labelled nucleotide building block is liberated from the nucleic acid molecule. In certain embodiments, it is also possible to carry out a sequence determination during elongation and degradation, i.e., when using nucleotide building blocks, which both carry a fluorescence-labelling group remaining with the building block and a fluorescence-labelling group which is cleaved off from the building block when the building block is incorporated into a nucleic acid molecule. In this embodiment, both fluorescent groups may be the same or different.

In some embodiments, the method involves one or more cycles of nucleic acid-synthesis and nucleic acid-degradation in order to determine the base sequence of a nucleic acid molecule template. The nucleic acid synthesis involves an elongation of the primer annealed to the nucleic acid template molecule catalyzed by the nucleic acid-synthesizing enzyme molecule, wherein a nucleic acid molecule complementary to the sequence of the nucleic acid template is generated. In the next step, the generated nucleic acid molecule is degraded by a nucleic acid-degrading enzyme molecule.

When a nucleotide building block is incorporated into an elongated nucleic acid molecule, a time dependent change in the fluorescence may occur, which can be detected as indicated above. Preferably, the incorporation of the nucleotide building blocks into the elongated nucleic acid molecule is associated with a detectable increase in the fluorescence, preferably with a transient increase in the fluorescence. For example, nucleotide building blocks may be used which carry a fluorescent labelling group on the portion of the molecule which is cleaved off when the building block is incorporated into the primer, e.g., on the γ-phosphate group.

When a nucleotide building block is cleaved off from the synthesized nucleic acid molecule, a time-dependent change of fluorescence may be determined due to the interaction of fluorescent labelling groups incorporated in nucleic acid strands with neighbouring groups, for example with chemical groups of the nucleic acids, in particular nucleobases such as, for example, G, or/and neighbouring fluorescent labelling groups, and these interactions leading to a change in fluorescence, in particular in fluorescence intensity, compared to the fluorescent labelling groups in "isolated" form, owing to quenching processes or/and energy transfer processes. The removal by cleavage of individual nucleotide building blocks alters the overall fluorescence, for example the fluorescence intensity of an immobilized nucleic acid strand, and this change is a function of the removal by cleavage of individual nucleotide building blocks, i.e., a function of time.

In certain embodiments, association of a labelled nucleotide with the biomolecule complex is detected by measuring polarisation of the emitted photons. The polarisation of excited states' photons is changed by the rotational movement of the light emitting nucleotide labels and can be used for identifying free moving contra bound labelled nucleotides in the polymerisation process.

This time-dependent change in fluorescence during elongation and/or degradation may be recorded in parallel for a multiplicity of nucleic acid molecules and correlated with the base sequence of the individual nucleic acid strands. Preference is given to using those fluorescent labelling groups which, when incorporated in the nucleic acid strand, are, at least partially, quenched so that the fluorescence intensity is increased after the nucleotide building block containing the labelling group or a neighbouring building block causing quenching has been removed by cleavage.

During incorporation and/or removal of individual nucleotide building blocks, it is possible to measure a change in fluorescence intensity of the nucleic acid strand or/and the incorporated or cleaved-off nucleotide building block, owing to quenching processes or energy transfer processes. This change in fluorescence intensity with time depends on the base sequence of the nucleic acid strand studied and can therefore be correlated with the sequence.

The complete sequence of the nucleic acid molecule may be determined by using a mixture of nucleotide building blocks, labelled on all four different bases, for example on A, G, C and T, or on combinations of two or three different bases. It is possible, where appropriate, to attach to the nucleic acid strand to be studied also a "sequence identifier", i.e., a labelled nucleic acid of known sequence, for example by enzymatic reaction using ligase or/and terminal transferase, so that at the start of sequencing initially a known fluorescence pattern and only thereafter the fluorescence pattern corresponding to the unknown sequence to be studied is obtained.

The detection comprises irradiating primary electromagnetic radiation from a radiation source into the support, preferably by means of a laser, or by another suitable light source, in order to cause excitation of the fluorescent labelling groups. In certain embodiments, the radiation source comprises a plurality of different lasers emitting radiation at different wavelengths. It is possible, in this connection, to use one or more laser beams, for example an expanded laser beam, having a cross section of approx. 1-20 mm, and/or multiple laser beams. The detection preferably comprises a multipoint fluorescence excitation by lasers, for example a dot matrix of laser dots generated via diffraction optics (cf. WO 2002/097406) or a quantum well laser.

Fluorescence emission of a plurality of nucleic acid strands may be detected in parallel using a detecting means, e.g., a detector matrix which comprises, for example, an electronic detector matrix, for example a CCD camera, a CMOS detector matrix, e.g., a CMOS camera, or an avalanche photodiode matrix. The detection may be carried out in such a way that fluorescence excitation and detection are carried out in parallel on a part or all nucleic acid strands studied. Preference is given to carrying out the detection on fluorescence light which is emitted essentially orthogonally from the support surface through the reaction space or through the support body.

The detection may be carried out, for example, by means of a single molecule detection, for example by fluorescence correlation spectroscopy, which involves exposing a very small, preferably confocal, volume element, for example from 10⁻²¹ to 10⁻¹⁰ I, to the excitation light of a laser, or another suitable light source, which light excites the receptors present in this measuring volume so that the latter emit fluorescence light, the fluorescence light emitted from said measuring volume being measured by means of a photodetector and the change in the measured emission with time being correlated with the concentration of the analyte, so that it is possible to identify, at an appropriately high dilution, individual molecules in said measuring volume. Details of the procedure and of the apparatus used for detection can be found in the disclosure of EP 0 679 251, the content of which is herein incorporated by reference. The confocal determination of single molecules is furthermore described in Rigler and Mets (Soc. Photo-Opt. Instrum. Eng. 1921 (1993), 239 ff.) and Mets and Rigler (J. Fluoresc. 4 (1994) 259-264), the contents of which are herein incorporated by reference.

Alternatively, or additionally, detection may also be carried out by way of time-resolved decay measurement, called "time gating", as described, for example, by Rigler et al., "Picosecond Single Photon Fluorescence Spectroscopy of Nucleic Acids", in: "Ultrafast Phenomena", D.H. Auston, Ed., Springer 1984, the content of which is herein incorporated by reference. Here, the fluorescent molecules are excited in a measuring volume followed by, e.g., at a time interval of ≥ 100 ps, opening a detection interval on the photodetector. In this way it is possible to keep background signals generated by Raman effects sufficiently low so as to enable single molecules to be detected in an essentially interference-free manner.

Further, the present disclosure is explained in detail by reference to the following specific embodiments.

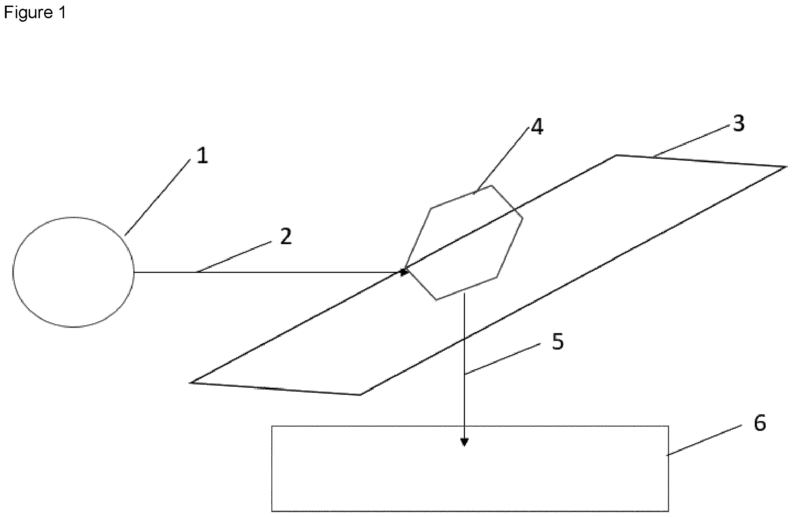

Fig. 1 shows an embodiment of the prior art. A radiation source (1) directs constant primary electromagnetic radiation (2) towards a molecule (4) bound to a support (3). The molecule (4) immobilized on the support (3) undergoes a bio-molecular process involving several states. Some of these states give rise to changes associated with the emission of secondary electromagnetic radiation (5) in response to irradiation by the constant primary electromagnetic radiation (2). Certain bio-molecular states cause unique emission profiles of secondary electromagnetic radiation (5) that is detected by a detector (6).

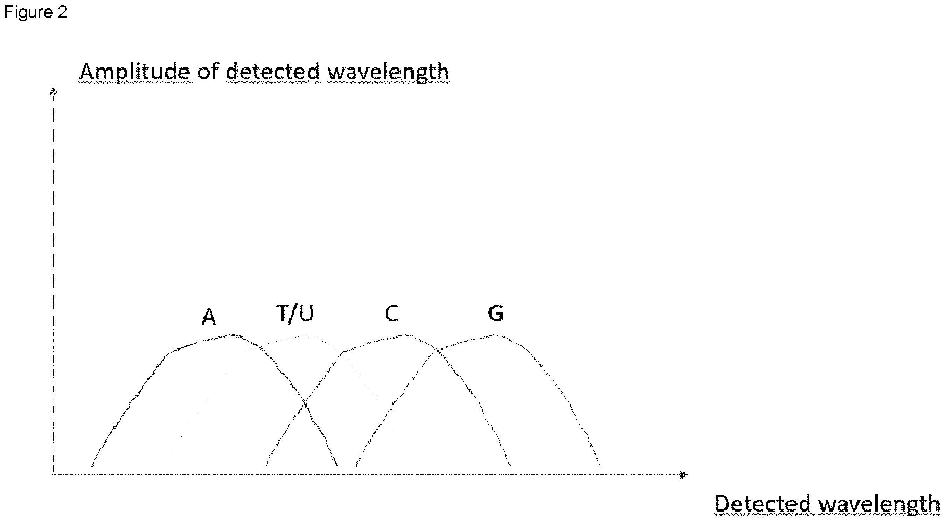

Fig. 2 shows an embodiment of the prior art. Single molecule DNA or RNA sequencing may comprise generating a secondary DNA or RNA strand that is complementary to a primary DNA or RNA strand. An enzyme, for example a polymerase, catalyzes the elongation of the secondary strand in that nucleotides, complementary to nucleotides in the primary strand, are incorporated sequentially one-by-one. Each nucleotide is fluorescent with a unique emission wavelength distribution. These emission distributions overlap to a certain extent which causes a limiting factor in the ability to accurately determine which nucleotide has been incorporated. The accuracy of single-molecule DNA or RNA sequencing consequently reaches a barrier.

Fig. 3 shows an embodiment of the present disclosure. The radiation source of the primary electromagnetic radiation comprises a set of 3 lasers having different wavelengths. During a molecular event, e.g. the incorporation a single nucleotide into an elongated secondary nucleic acid strand, the primary electromagnetic radiation changes in real time during a molecular event. This, in turn, gives rise to a detection of secondary electromagnetic radiation that can be plotted in a three dimensional graph. Since three dimensions are now available as compared with prior art embodiment according to Fig 2, there is no longer an overlap between secondary elctromagnetic radiation profiles of individual nucleotides which leads to an increased accuracy as compared with the prior art.

During the molecular event of an incorporation of A, T, G, or C, the radiation source switches between three laser wavelengths. Each of A, T, G, and C is attached to a fluorescent label, each with a characteristic excitation and emission wavelength distribution. Each of the three different laser wavelengths creates a unique response in terms of an emission from the fluorescent label. Based thereon, a three dimensional response can be constructed as shown. Since three dimensions are used, instead of a single dimension according to the prior art (Fig. 2), a much higher accuracy is achieved when deciding which of A, T, G, or C that was incorporated. The limited space in which each label provides its response is called the "fingerprint" of each of A, T, C, and G, respectively.

In this particular embodiment the individual lasers are turned on and off such the one laser is active at each given time. The lasers are activated in consecutive order in time. In a more general embodiment, a plurality of lasers, e.g., 2, 3, or 4 lasers can be activated in combinations of two, three of more in order to achieve the best accuracy which is when the distance between the "fingerprints" is the greatest.

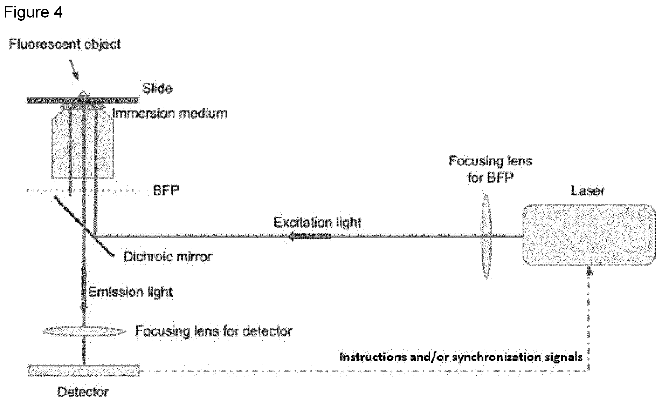

Fig. 4 shows an embodiment of the present disclosure. The detector and the source of radiation form a control system wherein the detector may control the source of radiation.

The detector (in some embodiments after consultation with hardware and/or software responsible for the analysis of the detected secondary electromagnetic radiation) provides via instruction and/or synchronization signals to the source of primary electromagnetic radiation (laser). These instructions may be, for example, to adjust, e.g., increase exposure time of a certain wavelength.

In certain embodiments, the detector and the source of primary electromagnetic radiation synchronize the changes made by the source of electromagnetic radiation amplitudes, wavelength, and/or other spectroscopic characteristics of the radiation source. In one embodiment, the detector controls exactly when, and which wavelengths, shall be emitted at any particular time by the radiation source. In another embodiment the detector's instructions to the source of electromagnetic radiation depend on the signal that was detected by the detector previously.

In a further embodiment the detector controls the source of radiation in real time based on data recorded during a molecular event. Hence, the detector has software or hardware, or is connected to a computer running software, that analyses the output signal and modifies the instructions to the detector during the analysis in order to optimize the accuracy of the determination of relevant parameters during a particular molecular event. Such modification can be, for example, to change the time during which a laser is turned on and off.

## Claims

1. A method for analyzing a single molecule event comprising:
- emitting a primary electromagnetic radiation from a radiation source to a sample where a single molecule event takes place, wherein said primary radiation causes emission of a secondary electromagnetic radiation from a component of said single molecule event, and wherein said secondary electromagnetic radiation is distinguishable from said primary electromagnetic radiation, and
- detecting said secondary electromagnetic radiation,
wherein the method further comprises altering at least one characteristic of said primary electromagnetic radiation during the time period of the single molecule event, whereby primary electromagnetic radiation having a first characteristic and primary electromagnetic radiation having at least one further characteristic is emitted to said sample during said time period wherein said first characteristic is different from said at least one further characteristic

2. The method of claim 1, wherein said secondary electromagnetic radiation is separately detected for said first characteristic and for said at least one further characteristic of said primary electromagnetic radiation and wherein a combined signal of said secondary electromagnetic radiation for said first characteristic and for said at least one further characteristic of said primary electromagnetic radiation is provided.

3. The method of claim 2, wherein said combined signal is a fingerprint from n different characteristics of said primary electromagnetic radiation, wherein n is particularly 2, 3 ,or 4.

4. The method of any one of the preceding claims, wherein said radiation source comprises at least one laser, particularly a plurality of lasers having different emission wavelengths.

5. The method of any one of the preceding claims, wherein said at least one sample comprises at least one sample spot on a support, wherein said support comprises a substrate and a plurality of sample spots on the surface of the support, and wherein the sample spots are spatially separated from another,
wherein a component of said single molecule event is immobilized to said at least one sample spot, wherein said immobilized component particularly comprises a biomolecule, e.g., a nucleic acid-polymerizing enzyme, particularly a DNA or RNA polymerase, and
wherein said at least one sample comprises at least one luminescent component of the single molecule event, e.g., a reactant, reaction intermediate and/or reaction product, wherein said at least one luminescent component emits said secondary electromagnetic radiation.

6. The method of claim 5, wherein the at least one luminescent component is a fluorescent component.

7. The method of claim 5 or 6, wherein said sample comprises a plurality of different luminescent components, particularly fluorescent components wherein at least some of said luminescent components, particularly fluorescent components have distinguishable and partially overlapping luminescence emission spectra, particularly distinguishable and partially overlapping fluorescence emission spectra.

8. The method of any one of the preceding claims comprising analyzing multiple subsequent single molecule events in one sample, particularly comprising separately analyzing multiple subsequent single molecule events in different samples in parallel.

9. The method of claim 8, wherein said multiple single molecule events comprise subsequent steps of a single molecule nucleic acid sequence analysis comprising multiple steps of nucleic acid elongation, wherein a luminescent nucleotide building block is incorporated into a nucleic acid molecule in the presence of a nucleic acid-polymerizing enzyme, and secondary electromagnetic radiation from the incorporation of said luminescent nucleotide building block into said nucleic acid molecule is detected.

10. The method of any one of the preceding claims comprising altering a characteristic of said primary electromagnetic radiation several times during the time period of the single molecule event.

11. The method of any one of the preceding claims comprising altering a characteristic of said primary electromagnetic radiation selected from the group:
- wavelength,
- amplitude,
- pulsed operation,
- polarization, and
- a combination of two or more of said characteristics.

12. The method of any one of the preceding claims comprising altering the characteristic of said primary electromagnetic radiation in time intervals that are in the range of about 50 ns to about 10 s, about 1 µs to about 500 ms, or about 10 µs to about 100 ms.

13. A device for analyzing a single molecule event comprising:
- means for providing at least one sample adapted for a single molecule reaction,
- a radiation source adapted for emitting a primary electromagnetic radiation to said sample, wherein said primary radiation causes emission of a secondary electromagnetic radiation from a component of said single molecule event, and wherein said secondary electromagnetic radiation is distinguishable from said primary electromagnetic radiation, and
- means for detecting said secondary electromagnetic radiation,
wherein the radiation source is adapted for altering at least one characteristic of said primary electromagnetic radiation at least once during the time period of the single molecule event, whereby primary electromagnetic radiation having a first characteristic and primary electromagnetic radiation having at least one further characteristic is emitted to said sample during said time period wherein said first characteristic is different from said at least one further characteristic.

14. The device of claim 13 adapted for performing the method of any one of claims 1-12.

15. Use of the method of any one of claims 1-12 or the device of any one of claims 13-14 for providing an increased accuracy in the analysis of a single molecule event.
